# EUROPÄISCHE PATENTSCHRIFT

(11) **EP 1 190 674 B1**
(45) Veröffentlichungstag und Bekanntmachung des Hinweises auf die Patenterteilung: **10.10.2007**
(21) Anmeldenummer: 01122922.6
(22) Anmeldetag: 25.09.2001
(51) Int. Cl.: A61B 5/155

(54) **Lanzettensystem**
Lancet devices
Dispositif de lancettes

(30) Priorität: 26.09.2000 DE 10047419
(43) Veröffentlichungstag der Anmeldung: 27.03.2002
(73) Patentinhaber: Roche Diagnostics GmbH, 68305 Mannheim (DE); F. HOFFMANN-LA ROCHE AG, 4070 Basel (CH)
(72) Erfinder: Fritz, Michael, 68647 Bilbis (DE); List, Hans, 64754 Hasseneck-Kailbach (DE); Weiss, Thomas, 68307 Mannheim (DE); Deck, Frank, 67150 Niederkirchen (DE); Argauer, Herbert, 92712 Pirk (DE); Immekus, Claudio, 68307 Mannheim (DE)

(56) Entgegenhaltungen:
- DE-B- 2 803 345
- US-A- 5 514 152
- US-A- 5 578 014
- US-A- 6 036 924

## Beschreibung

Die Erfindung betrifft ein System zum Bevorraten und Bereitstellen von Lanzetten zur Gewinnung von Körperflüssigkeiten, insbesondere zur Blutgewinnung.

Die Untersuchung von Proben von Körperflüssigkeiten, insbesondere von Blutproben, ermöglicht in der klinischen Diagnostik das frühzeitige und zuverlässige Erkennen von pathologischen Zuständen sowie die gezielte und fundierte Kontrolle von Körperzuständen. Die medizinische Blutdiagnostik setzt stets die Gewinnung einer Blutprobe des zu untersuchenden Individuums voraus. Während in Kliniken und bei niedergelassenen Ärzten oftmals durch eine Venenpunktion mehrere Milliliter Blut einer zu untersuchenden Person für die Analyse gewonnen werden, um damit eine Vielzahl von Labortests durchführen zu lassen, reichen für einzelne Analysen, die gezielt auf einen Parameter gerichtet sind, heutzutage oftmals wenige Mikroliter Blut oder sogar noch weniger aus. Solch geringe Blutmengen erfordern keine Venenpunktion. Vielmehr genügt es hier, zur Blutgewinnung durch die Haut z. B. in die Fingerbeere oder das Ohrläppchen der zu untersuchenden Person mit Hilfe einer sterilen, scharfen Lanzette zu stoßen, um so einige wenige Mikroliter Blut oder sogar Blutmengen im Nanoliterbereich für die Analyse zu gewinnen. Insbesondere eignet sich diese Methode, wenn die Analyse der Blutprobe unmittelbar nach der Blutgewinnung durchgeführt werden kann.

Vor allem im Bereich des sogenannten "Home-Monitoring", also dort, wo medizinische Laien einfache Analysen des Bluts selbst durchführen, und dort insbesondere für die regelmäßige, mehrmals täglich durchzuführende Blutgewinnung durch Diabetiker für die Kontrolle der Blutglucosekonzentration, werden Lanzetten und dazu passende Geräte (sogenannte Blutentnahmegeräte, Blutlanzettenvorrichtungen oder Stechhilfen), angeboten, die eine möglichst schmerzarme und reproduzierbare Blutgewinnung ermöglichen. Als Beispiele für Lanzetten und Stechhilfen seien die kommerziell erhältlichen Geräte (Stechhilfen) und Lanzetten Glucolet^{®} der Bayer AG und Softclix^{®} der Roche Diagnostics GmbH genannt.

Solche Lanzetten und Geräte (Stechhilfen) sind Gegenstand beispielsweise von EP-A 0 565 970.

Bei den derzeit kommerziell verfügbaren Systemen erfolgt die Bereitstellung der Lanzetten für die Verwendung in Stechhilfen meist in loser Form. Der Benutzer entnimmt manuell vor jedem Stechvorgang eine Lanzette aus einer Verpackung, beispielsweise einer Pappschachtel oder einer Röhre, in der eine Vielzahl von Lanzetten (meist ungeordnet, lose geschüttet) enthalten ist. Anschließend wird die Stechhilfe, beispielsweise durch Abschrauben oder Abziehen einer Kappe, für die Aufnahme der Lanzette vorbereitet, wobei der Lanzettenhalter der Stechhilfe freigelegt wird. Die aus der Packung entnommene Lanzette wird manuell in den Lanzettenhalter der Stechhilfe eingeführt und dort fixiert. Dann muss die Schutzhülle von der Lanzette manuell abgenommen werden. Anschließend wird die Stechhilfe mit ihrer Kappe wieder verschlossen. Die Kappe sorgt dafür, dass die Lanzette von außen nicht mehr zugänglich ist. Sie besitzt meist eine Öffnung, durch welche die Lanzettenspitze beim eigentlichen Stechvorgang austreten kann. Schließlich wird die Stechhilfe gespannt und steht für den Stechvorgang zur Gewinnung von Blut zur Verfügung.

Die Vielzahl der manuellen Bedienschritte bei herkömmlichen Lanzettensystemen (Lanzette und Stechhilfe) wird vom Benutzer als nachteilig empfunden und ist vor allem bei eingeschränkter Wahrnehmung im Zustand einer Hypoglykämie problematisch. Zudem wird der Benutzer nicht daran gehindert, eine einmal eingelegt Lanzette mehrfach zum Stechen und Blutgewinnen zu verwenden. Das mehrfache Benutzen einer Lanzette hat zum einen aus hygienischen Gründen zu unterbleiben, insbesondere wenn mehr als eine Person das Lanzettensystem benutzt, wie dies beispielsweise in Arztpraxen oder Krankenhäusern der Fall sein kann oder wenn nicht ausgeschlossen werden kann, dass eine Fehlbenutzung z. B. durch Kinder erfolgt. Zum anderen führt die mehrmalige Benutzung der Lanzetten zu steigendem Schmerz für den Benutzer, denn da die Lanzetten für den einmaligen Gebrauch konzipiert sind, werden sie bei mehrmaligem Gebrauch schnell stumpf. Zudem besteht mit den Stechhilfen und Lanzetten des Standes der Technik die Gefahr, dass die Lanzetten unsachgemäß in die Stechhilfen eingelegt werden. Weiterhin kann sich ein Benutzer bei unsachgemäße Benutzung von Lanzetten und Stechhilfen unbeabsichtigt verletzen.

Es mangelt deshalb nicht an Versuchen, die angesprochenen Nachteile zu beseitigen. Aus US 5,514,152, US 5,152,775, US 4,794,926 und US 5,035,704 sind Stechhilfen bekannt, die mehrere Lanzetten in sich bevorraten und diese nacheinander einzeln für Stechvorgänge benutzen können. Nach dem Stechvorgang können die Lanzetten einzeln aus dem Gerät entfernt werden. Die Magazinierung und die mögliche automatisierte Bereitstellung der Lanzetten helfen, Fehler beim Einlegen einer Lanzette in eine Stechhilfe zu vermeiden.

Zur Durchführung der Stechbewegung muss die Lanzette im Blutentnahmegerät (Stechhilfe) zunächst in eine Stechrichtung (das heißt mit ihrer Spitze auf die zu durchstoßende Körperpartie hin) bewegt oder ausgelenkt werden und anschließend möglichst schnell in ihre Ausgangslage zurückgeholt werden.

In herkömmlichen Lanzettensystemen, wie sie beispielsweise in EP-A 0 565 970 oder in DE 2803345 beschrieben sind, wird zu diesem Zweck die Lanzette in ihrem der Spitze abgewandten Teil möglichst fest von einem Lanzettenhalter, der Teil der Stechhilfe ist, umschlossen und sowohl in Stechrichtung (vorwärts) als auch zurück in die Ausgangsposition (rückwärts) bewegt. Die Vor- und Rückwärtsbewegung wird über einen einzigen Antriebsmechanismus realisiert, der oftmals federgetrieben ist.

Ein automatischer Lanzettenwechsel, beispielsweise unter Verwendung eines Lanzettenmagazins, ist mit einem solchen System, bei dem ein Umschließen der Lanzette durch einen Lanzettenhalter erforderlich ist, nur schwer zu realisieren. Zudem muss die Lanzette über einen an den Lanzettenhalter angepassten Lanzettenkörper verfügen, der eine feste Verbindung zwischen Lanzette und Halter herstellt, insbesondere um die Rückwärtsbewegung sicherzustellen. Die Lanzetten sind folglich oft voluminös und ihre Bevorratung in einem Magazin würde zu großen Dimensionen des Lanzettensystems führen.

Das in US 4,794,926 beschriebene Lanzettensystem nutzt sowohl zur Vor- als auch zur Rückwärtsbewegung der Lanzette die Federkraft einer Blattfeder aus, die Teil der Lanzette ist. Dieses Antriebskonzept ist jedoch für im wesentlichen nadelförmige Lanzetten nicht geeignet.

Aus US 5,578,014 ist ein Lanzettensystem bekannt, bei dem der Antrieb der Lanzette für die Vorwärtsbewegung unabhängig vom Antrieb für die Rückwärtsbewegung arbeitet. Die Vorwärtsbewegung wird durch einen federgetriebenen Stößel bewirkt, der Teil einer Stechhilfe ist und auf die Lanzette von hinten, das heißt von ihrer der Spitze abgewandten Seite, einwirkt. Die Rückwärtsbewegung wird durch eine Feder, die in der Lanzette enthalten ist, angetrieben. Ähnliche Systeme werden in US 5,029,583 und DE-A 198 55 465 sowie US 6,036,924 beschrieben. Nachteilig an dieser Variante ist, dass jede Lanzette mit einer eigenen Feder ausgerüstet sein muss, was ihre Herstellung kompliziert und teuer macht. Zudem ist eine Miniaturisierung des Systems nur schwer möglich.

Der Versuch, die Herstellkosten für derartige Lanzetten, die ja Massenartikel zum Einmalgebrauch (sog. Disposables) sind, zu senken führt oftmals dazu, dass kostengünstige Rückholfedern eingesetzt werden müssen. Oftmals sind die Materialeigenschaften in diesem Fall großen Schwankungen unterworfen, was dazu führt, dass auch die Rückholgeschwindigkeit erheblichen Schwankungen unterworfen ist. Dies kann sich negativ auf das Schmerzempfinden beim Stechvorgang auswirken.

Aufgabe der Erfindung ist es daher, die Nachteile des Standes der Technik zu beseitigen. Insbesondere ist es Aufgabe der vorliegenden Erfindung, ein System zur Gewinnung von Blut oder anderen Körperflüssigkeiten bereitzustellen, welches eine Vielzahl von Lanzetten auf möglichst kleinem Raum bereitstellt.

Diese Aufgabe wird durch den Gegenstand der Erfindung gelöst.

Gegenstand der Erfindung ist ein System zum Bevorraten und Bereitstellen von Lanzetten zur Gewinnung einer Körperflüssigkeit, insbesondere zur Blutgewinnung gemäß Anspruch 1, sowie ein Lanzettenmagazin gemäß Anspruch 6, und eine Stechhilfen gemäß Anspruch 10. Bevorzugte Ausführungsformen der Erfindung werden durch die abhängigen Ansprüche definiert.

Das erfindungsgemäße System umfasst
i) eine Vielzahl von im wesentlichen nadelförmigen Lanzetten;
ii) eine Antriebseinheit, die ein Antriebselement, beispielsweise einen Stößel, besitzt, der zur Ausführung eines Stechvorgangs aus einer Ruheposition in eine Stechposition bewegt wird;
iii) einen Aufbewahrungsbereich zum Aufbewahren der Lanzetten;
iv) einen Entnahmebereich zum Herausführen zumindest der Spitze einer Lanzette aus dem System während des Stechvorgangs; und
v) eine Transporteinheit, die Lanzetten aus dem Aufbewahrungsbereich in den Entnahmebereich transportieren kann. Das Antriebselement ist im erfindungsgemäßen System so angeordnet, dass es im Entnahmebereich auf die dort befindliche Lanzette zur Ausführung des Stechvorgangs einwirken kann, beispielsweise wie ein Hammer oder ein Schlagbolzen auf ein Ende der Lanzette schlagend, und diese so aus ihrer Ruheposition in eine Stechposition bewegt wird. Ferner weist das System im Entnahmebereich eine Rückholvorrichtung auf, die mit der Lanzette, die sich im Entnahmebereich befindet,
   wechselwirken kann, um die Lanzette nach der Ausführung des Stechvorgangs von der Stechposition in ihre Ruheposition zu bewegen. Die Antriebseinheit und die Rückholvorrichtung greifen hierbei unabhängig voneinander an der Lanzette an, wobei die Rückführung der Lanzette durch eine zentrale Rückholvorrichtung bewirkt wird.

Das erfindungsgemäße System umfasst eine Vielzahl von - das heißt mindestens zwei - im wesentlichen nadelförmigen Lanzetten. "Im wesentlichen nadelförmig" bedeutet dabei, dass die Lanzetten eine metallene Lanzettennadel mit einer Spitze aufweisen, die gegebenenfalls angeschliffen sein kann. Wichtig ist dabei, dass die Lanzette selbst keine Feder oder ähnliches aufweist, die sie nach der Stechbewegung in ihre Ausgangslage zurückführt. Die Rückführung der Lanzette wird durch eine zentrale Rückholvorrichtung im System bewirkt.

Zur leichteren Handhabung der Lanzette im System, beispielsweise zum Transport der Lanzette aus ihrer Aufbewahrungsposition in die Entnahmeposition oder zum Greifen der Lanzette durch die Rückholvorrichtung, kann an die Lanzettennadel ein Kunststofflanzettenkörper aus einem spritzgussfähigen Material angespritzt sein. Um das Greifen der Lanzette durch die Rückholvorrichtung zu erleichtern kann die Lanzettennadel an ihrem stumpfen, das heißt der Spitze abgewandten Ende auch gestaucht, umbördelt, geknickt oder eingekerbt sein.

Der Lanzettenkörper ist meist ein Kunststoffbauteil, das um die Lanzettennadel herum angebracht ist und diese umschließt. Der Lanzettenkörper erleichtert das Greifen der Lanzettennadel und kann auf einfache Weise eine spezielle, auf die Geometrie der Stechhilfe angepasste Form aufweisen. Die äußere Gestalt der Lanzette wird so im Wesentlichen vom Lanzettenkörper bestimmt.

Die Spitze der Lanzettennadel ist - im unbenutzten Zustand - zur Sicherstellung ihrer Sterilität vorzugsweise von einer Schutzhülle umgeben. Diese kann aus demselben Material wie der Lanzettenkörper bestehen und bildet dann meist mit diesem eine Einheit. Die Schutzhülle kann vor der Benutzung der Lanzette vom Lanzettenkörper getrennt und von der Spitze der Lanzettennadel abgenommen werden. Zwischen Lanzettenkörper und Schutzhülle befindet sich zu diesem Zweck vorzugsweise eine Sollbruchstelle.

Die Lanzette der Erfindung beinhaltet eine Nadel (Lanzettennadel) mit einer Spitze. Die Nadel ist in der Regel mehrere Millimeter (mm) bis wenige Zentimeter (cm) lang und weist eine längliche Gestalt auf. Typischerweise besitzen Nadeln eine zylindrische Gestalt, da diese Nadelform besonders gut herstellbar ist; es sind jedoch auch Nadelformen mit abweichender Formgebung möglich. Der Spitzenbereich der Nadel beinhaltet die Nadelspitze, die beim bestimmungsgemäßen Gebrauch der Lanzette in Gewebe eingestochen wird. Die Spitze der Lanzettennadel ist folglich der Teil der Lanzette, der mit der Haut des zu stechenden Individuums in Berührung kommt, diese ggf. verletzt und so den Ausfluss einer Körperflüssigkeit, insbesondere Blut oder interstitielle Flüssigkeit, verursacht.

Die Spitze der Lanzettennadel kann beispielsweise rotationssymmetrisch sein, wie dies im Allgemeinen bei Nähnadeln der Fall ist. Es hat sich jedoch als vorteilhaft herausgestellt, wenn man an der Nadelspitze einen oder mehrere Schliffe anbringt. Die hierbei entstehenden, zur Längsachse der Nadel geneigten, in einer Spitze zulaufenden Kanten dienen beim Einstich als scharfe Schneide und gestalten den Einstichvorgang schmerzärmer, als dies mit rotationssymmetrischen Nadeln der Fall ist.

Die Lanzettennadel der erfindungsgemäßen Lanzette ist aus einem Material gefertigt, das ausreichend hart ist, um eine mechanische Beanspruchung während des Einstichvorgangs, der Bearbeitungsschritte bei ihrer Herstellung oder eventuell sonstige auftretende Beanspruchungen ohne Deformation zu überstehen. Weiterhin muss das Material so beschaffen sein, dass während des Einstichvorgangs keine Partikel abbrechen oder sich ablösen. Schließlich muss das Nadelmaterial auch so bearbeitbar sein, dass die Nadelspitze ausreichend spitz und die Kanten der Nadelspitze gegebenenfalls ausreichend scharf geschliffen werden können. Gut geeignete Materialien für die Lanzettennadel sind vor allem Metalle und von diesen insbesondere Edelstähle. Es sind jedoch auch Nadeln aus Keramik, Silizium oder Kunststoffen denkbar. Edelstahlnadeln sind besonders bevorzugt.

In einer bevorzugten Ausführungsform ist zumindest die Spitze der Lanzettennadel der erfindungsgemäßen Lanzette von einem Kunststoffkörper, der im folgenden als Lanzettenkörper bezeichnet werden soll, umgeben. Bevorzugt ist dabei, dass der Lanzettenkörper im Bereich der Spitze der Lanzettennadel aus einem elastischen Material besteht. Zumindest die Spitze der Lanzettennadel ist von diesem elastischen Material allseitig vollständig umgeben, das heißt in dieses eingebettet, und so von der Umgebung abgeschlossen. Das elastische Material des Lanzettenkörpers, welches in unterschiedlichen Ausführungsformen den Lanzettenkörper vollständig oder nur teilweise bilden kann, zeichnet sich dadurch aus, dass es weich, verformbar und von der Lanzettennadel mit ihrer Spitze durchstoßbar ist, ohne die Spitze zu verletzen. Beim Stechvorgang wird die Lanzettennadel entlang ihrer Längsachse relativ zum Lanzettenkörper bewegt und tritt mit ihrer Spitze aus dem Lanzettenkörper aus, um so zur Blutgewinnung in die Haut des zu untersuchenden Individuums einstechen zu können. Eine wichtige Eigenschaft ist ferner, dass sich das elastische Material gegebenenfalls beim Zurückziehen der Lanzettennadel in den Lanzettenkörper wieder um die Spitze der Lanzettennadel verschließt. Nach dem Stechvorgang kann die Lanzettennadel in einer bevorzugten Ausführungsform in Umkehrung der Bewegung beim Stechvorgang in ihre Ausgangslage relativ zum Lanzettenkörper gebracht werden, in der die Spitze wieder allseitig vollständig vom elastischen Material des Lanzettenkörpers umschlossen ist.

Das elastische Material des Lanzettenkörpers, welches die Spitze der Lanzettennadel vollständig umschließt, gewährleistet die Sterilität der Lanzettennadelspitze vor deren Benutzung, vorzugsweise bis unmittelbar vor deren Benutzung, und sorgt gegebenenfalls für ein Umschließen der Lanzettennadelspitze nach deren Benutzung. Das elastische Material ist folglich dicht für das Eindringen von Keimen. Zudem stellt das elastische Material einen mechanischen Schutz für die Lanzettennadelspitze dar und verhindert so auch ein unbeabsichtigtes Verletzen an der Lanzettennadelspitze.

Als elastisches Material für den Lanzettenkörper der vorliegenden Ausführungsform haben sich Gummi, Kautschuk, Silikone, Elastomere und insbesondere thermoplastische Elastomere als geeignet herausgestellt. Diese weisen die für die vorliegende Erfindung wesentlichen Eigenschaften auf: sie sind weich, verformbar, von der Lanzettennadel zu durchstoßen, ohne die Spitze zu verletzen, und sie schließen sich dicht um die benutzte Lanzettennadelspitze. Desweiteren können Sie für Spritzgussprozesse verwendet werden, die eine Massenfertigung von Lanzetten in großen Stückzahlen ermöglichen.

Während des Stechvorgangs wird die Lanzettennadel relativ zum Lanzettenkörper bewegt. Letzterer wird dabei vorzugsweise von der Stechhilfe oder dem Stechgerät in seiner Position fixiert. Die Lanzettennadel kann zum Zwecke ihres Antriebs in Vorwärts- und/oder Rückwärtsrichtung geeignete Elemente enthalten. Insbesondere kann sie besonders ausgeformt sein, beispielsweise einen Nadelkopf, eine Umbördelung, einen Knick oder eine Aussparung an dem der Spitze entgegengesetzten Ende besitzen, oder zusätzlich zum Lanzettenkörper, der die Spitze umschließt, einen weiteren Lanzettenkörper aufweisen, der von dem Antriebselement des Lanzettensystems ergriffen werden kann. Die Ausformung der Nadel oder der zusätzliche Lanzettenkörper können in geeigneter Weise mit einer entsprechenden Antriebsvorrichtung und/oder Rückholvorrichtung im Stechgerät (Stechhilfe) wechselwirken.

Zur Erhöhung der Stabilität des elastischen Materials ist es möglich, dieses mit einem steifen Material, beispielsweise einem steifen Kunststoffmaterial, zu verbinden. Das elastische Material kann dabei beispielsweise auf seiner Außenseite, die nicht mit der Spitze der Lanzettennadel in Berührung kommt, mit einer Schicht eines steifen Materials, beispielsweise eines steifen Kunststoffs, stabilisiert sein. Es ist auch möglich, den Lanzettenkörper nur im Bereich der Lanzettennadelspitze aus einem elastischen Material zu fertigen, in den übrigen Bereichen den Lanzettenkörper jedoch aus herkömmlichen, steifen Kunststoffen zu fertigen. Dabei können das elastische Material und das steife Material miteinander verklebt sein oder durch einen Spritzgussprozess, beispielsweise einen Zweikomponenten-Spritzgussprozess, miteinander verbunden sein. Das steife Material des Lanzettenkörpers sorgt dabei für eine mechanische Stabilisierung des elastischen Materials während des Stechvorgangs und erleichtert die Fixierung des elastischen Teils des Lanzettenkörpers während des Stechvorgangs im Lanzettensystem.

In einer weiteren bevorzugten Ausführungsform enthält die Lanzette eine Lanzettennadel mit einer Spitze und einen Hohlkörper, der zumindest die Spitze der Lanzettennadel umgibt, wobei bei der Lanzette die Lanzettennadel im Bereich ihrer Spitze im Hohlkörper beweglich ist und der Hohlkörper von einer Folie verschlossen ist, die von der Spitze der Lanzettennadel beim Stechvorgang durchstoßen werden kann und die sich gegebenenfalls nach dem Zurückziehen der Spitze der Lanzettennadel in den Hohlkörper wieder verschließt.

Vorteilhafterweise ist dieser Hohlkörper in den Bereichen, die nicht mit der Spitze der Lanzettennadel in Berührung kommen, aus einem steifen, vorzugsweise spritzgussfähigen Material gefertigt.

Beim Stechvorgang wird die Lanzettennadel relativ zum Hohlkörper, der den Lanzettenkörper darstellt, bewegt. Halterung und Antrieb der Lanzettennadel und Fixierung des Lanzettenkörpers können, wie oben beschrieben, durch geeignete konstruktive Maßnahmen realisiert werden.

Das elastische Material, welches einen Teil des hohlen Lanzettenkörpers ausmacht, wird beim Stechvorgang von der Lanzettennadelspitze durchstoßen und verschließt sich gegebenenfalls nach dem Zurückziehen der Lanzettennadelspitze in den Hohlkörper wieder und schließt so den Hohlkörper ab. Die Lanzettennadelspitze ist somit bis unmittelbar vor Gebrauch steril im Hohlkörper versiegelt und wird nach Gebrauch hygienisch in ihm eingeschlossen.

Die Lanzette dieser Ausführungsform kann neben dem Lanzettenkörper, der die Spitze der Lanzettennadel umschließt, einen weiteren Lanzettenkörper aufweisen, der im Zusammenspiel mit geeigneten Elementen einer Stechhilfe während des Stechvorgangs mit diesen wechselwirkt. Ebenso kann die Lanzettennadel speziell geformt sein, beispielsweise einen Kopf an dem der Spitze entgegengesetzten Ende besitzen, um den Antrieb der Lanzette zu erleichtern.

In einer bevorzugten Ausführungsform weist die im wesentlichen nadelförmige Lanzette an ihrem der Spitze gegenüberliegenden Ende einen Nadelkopf auf. Dieser kann im erfindungsgemäßen System mit der Rückholvorrichtung wechselwirken, so dass die Rückholvorrichtung die Lanzette nach dem Stechvorgang in die Ausgangsposition (Ruheposition) zurückbewegen kann. Der Nadelkopf kann - wie dies zum Beispiel bei Stecknadeln der Fall ist - ein integraler Teil der Lanzettennadel sein. Vorzugsweise ist die Lanzettennadel dann aus einem Stück, insbesondere aus einem Metall gefertigt. Es ist jedoch auch möglich, dass der Nadelkopf ein separates, mit der Nadel verbundenes Teil ist, beispielsweise ein an die Nadel angespritztes Kunststoffteil. Der Nadelkopf kann ein abgeflachtes, mit einem Wulst versehenes Ende der Lanzettennadel sein, analog beispielsweise dem Kopf einer Stecknadel oder der Schlagfläche eines Nagels. Alternativ kann der Nadelkopf eine endständige Verdickung der Lanzettennadel oder ein die Lanzettennadel umgebender und diese fest verschließender Ring oder eine auf das stumpfe Ende der Nadel aufgesetzte Scheibe sein.

Das erfindungsgemäße System enthält weiterhin eine Antriebseinheit für die Lanzette. Die Antriebseinheit enthält ein Antriebselement, beispielsweise einen Stößel oder einen Hammer, der zur Ausführung eines Stechvorgangs aus einer Ruheposition in eine Stechposition bewegt wird. Das Antriebselement ist im erfindungsgemäßen System so angeordnet, dass es im Entnahmebereich auf die dort befindliche Lanzette zur Ausführung des Stechvorgangs einwirken kann, beispielsweise wie ein Hammer oder ein Schlagbolzen auf ein Ende der Lanzette schlagend, und diese so aus ihrer Ruheposition in eine Stechposition bewegt wird. Wichtig ist im Zusammenhang mit der hier vorliegenden Erfindung, dass die Antriebseinheit zwar die Vorwärtsbewegung der Lanzette bewirkt, das heißt die Bewegung, welche die Lanzette aus ihrer Ruheposition in die Stechposition bringt, jedoch nicht ursächlich für die Rückwärtsbewegung der Lanzette ist. Die Antriebseinheit kann nach an sich bekannter Art und Weise ausgestaltet sein und beispielsweise über einen Federantrieb und gegebenenfalls Getriebeelemente verfügen. Solche Antriebselemente sind dem Fachmann im Prinzip bekannt und können ohne weiteres realisiert werden.

Geeignete Antriebe sind aus dem Stand der Tachnik in vielen Ausführungen bekannt. Der Antriebsstößel vollführt dabei eine Vorschub- und anschließend eine Rückzugsbewegung.

Während der Vorschubbewegung wird die Lanzette ebenfalls nach vorne, das heißt in ihre Stechrichtung, bewegt. Während der Rückzugsbewegung des Antriebselements wirkt dieses nicht länger auf die Lanzette ein. Dies erlaubt es der Lanzette, ebenfalls eine Rückwärtsbewegung auszuführen. Da die Lanzette erfindungsgemäß mit einer Rückholeinrichtung gekoppelt wird, kann diese auch die Rückwärtsbewegung des Antriebsstößels mit übernehmen. Dies ist z.B. dann sinnvoll, wenn der Antrieb ein einfacher Nocken ist, wie er aus Ventiltrieben von Motoren bekannt ist. Auch bei einfachen, federbelasteten Stößeln, die aus der gespannten Lage vorschnellen, und auf die Lanzette treffen und diese so vorwärts bewegen, ist das möglich. Im Fall einer zwangsgesteuerten Bewegung des Antriebselements, das heißt, wenn sowohl die Vorwärts- als auch die Rückholbewegung des Antriebselements durch einen geeigneten Antrieb realisiert wird, bewegt die Rückholeinrichtung dagegen nur die Lanzette.

Das erfindungsgemäße System dient insbesondere zur Aufbewahrung und automatisierten Zurverfügungstellung von Lanzetten für Stechvorgänge und der Ausführung der Stechvorgänge. Es weist deshalb auch einen Aufbewahrungsbereich zum Aufbewahren der Lanzetten auf. Beispielsweise kann das System einen Raum für die Aufnahme eines Lanzettenmagazins aufweisen. Im Aufbewahrungsbereich können zumindest die unbenutzten Lanzetten untergebracht werden. Vorzugsweise ist es auch möglich, dort die benutzten Lanzetten bis zu ihrer Entsorgung zu speichern. Das System kann auch getrennte Aufbewahrungsbereiche für unbenutzte und benutzte Lanzetten aufweisen.

Daneben weist das System der vorliegenden Erfindung einen Entnahmebereich zum Herausführen zumindest der Spitze einer Lanzette aus dem System während des Stechvorgangs auf. Zur Ausführung der Stechbewegung wird zunächst eine im System befindliche Lanzette aus dem Aufbewahrungsbereich in den Entnahmebereich transportiert. Dort befindet sich die Lanzette dann in einer Position, in der sie einerseits vom Antriebselement der Antriebseinheit in Vorwärtsrichtung, das heißt in Richtung der Stechbewegung, angetrieben werden kann, und in der sie andererseits mit der Rückholvorrichtung in geeigneter Weise wechselwirken kann, um nach dem Stechvorgang in ihre Ausgangsposition zurückgebracht zu werden. Durch geeignete konstruktive Maßnahmen ist sicherzustellen, dass die Lanzette im Entnahmebereich sowohl mit der Antriebseinheit, insbesondere mit ihrem Antriebselement, als auch mit der Rückholvorrichtung wechselwirken kann. Desweiteren weist das erfindungsgemäße System im Entnahmebereich eine Öffnung auf, die ein Austreten der Lanzettenspitze aus dem System während des Stechvorgangs ermöglicht.

Das erfindungsgemäße System besitzt als weitere Komponente eine Transporteinheit, die Lanzetten aus dem Aufbewahrungsbereich in den Entnahmebereich transportieren kann. Die Transporteinheit kann manuell oder automatisch getrieben und/oder gesteuert sein. Die genaue Art der Transporteinheit ist für die vorliegende Erfindung nicht wesentlich und kann je nach genauer Art der Lagerung der Lanzetten im System unterschiedlich sein. Die Transporteinheit kann manuell oder mit Hilfe einer Feder oder eines Motors angetrieben sein.

Ferner weist das System im Entnahmebereich eine zentrale Rückholvorrichtung auf, die mit der Lanzette, die sich im Entnahmebereich befindet, wechselwirken kann, um die Lanzette nach der Ausführung des Stechvorgangs von der Stechposition in ihre Ruheposition zu bewegen. Die Antriebseinheit und die Rückholvorrichtung greifen hierbei unabhängig voneinander an der Lanzette an. Die Rückholvorrichtung weist in einer bevorzugten Ausführungsform einen Federmechanismus auf, welcher besonders bevorzugt eine Blatt- oder Schraubenfeder umfasst. Alternativ kann die Rückholvorrichtung ein verformbares Elastomer besitzen, beispielsweise einen komprimierbaren Gummiblock, der sich bei Entlastung entspannt und so die Rückholbewegung bewirkt. Besonders bevorzugte Ausführungsformen für die Rückholvorrichtung sowie deren spezielle Wechselwirkung mit den Lanzetten werden im Zusammenhang mit den Figuren (siehe unten) beschrieben. Im Unterschied zum Stand der Technik weist das erfindungsgemäße System nur eine zentrale Rückholvorrichtung auf; das bedeutet, einzelne Lanzetten besitzen keine Rückholfeder oder dergleichen, die sie nach der Ausführung der Stechbewegung in die Ausgangslage zurückbewegen.

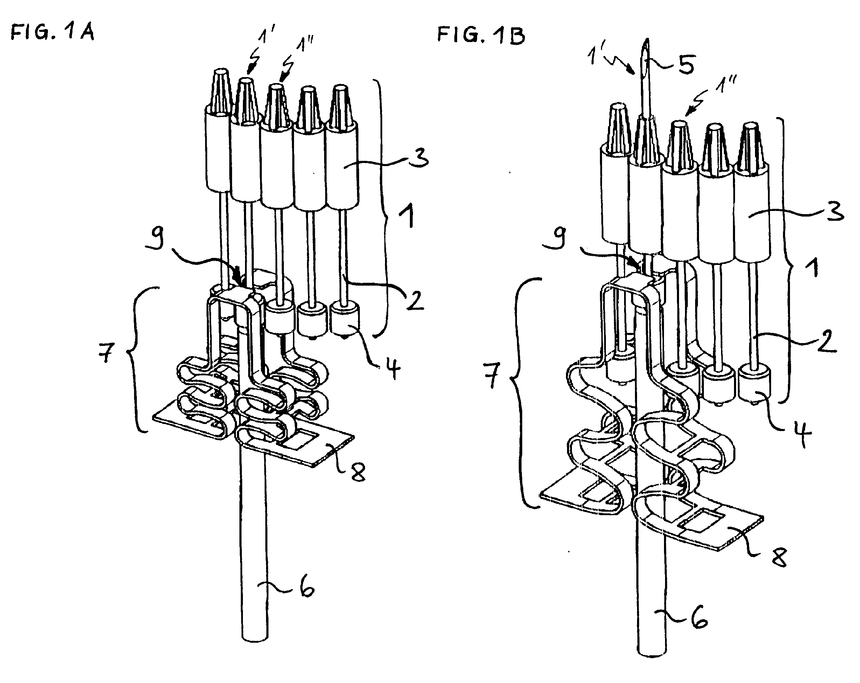

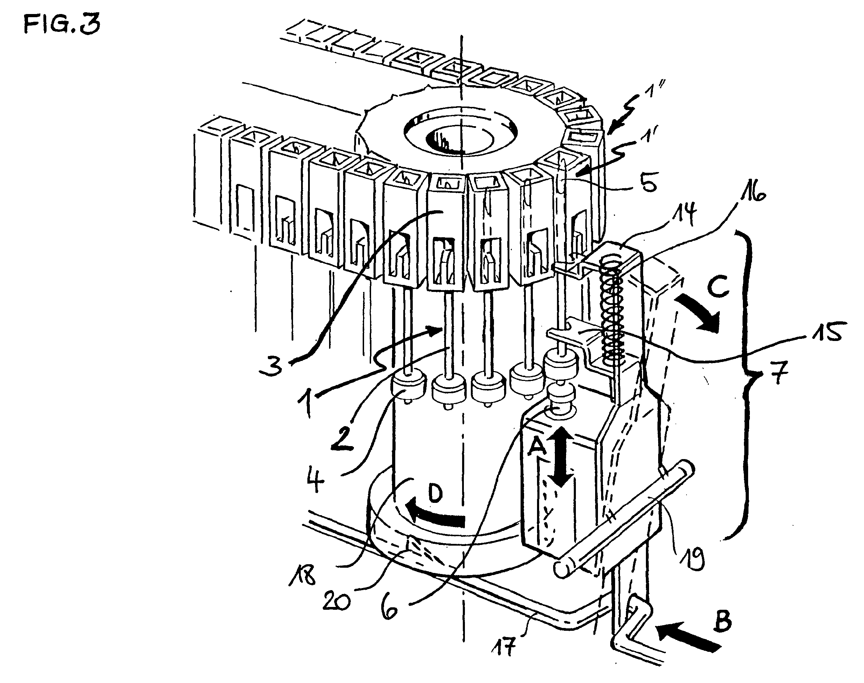

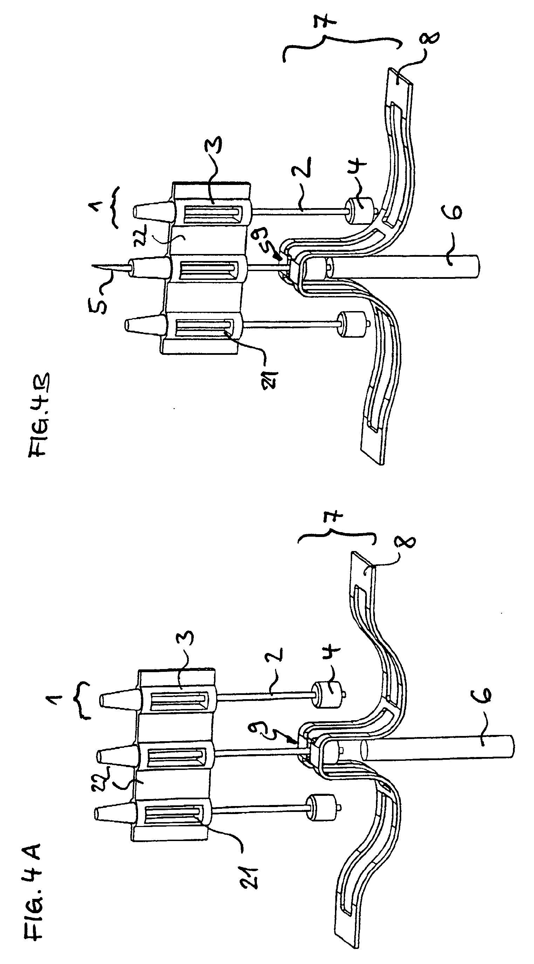

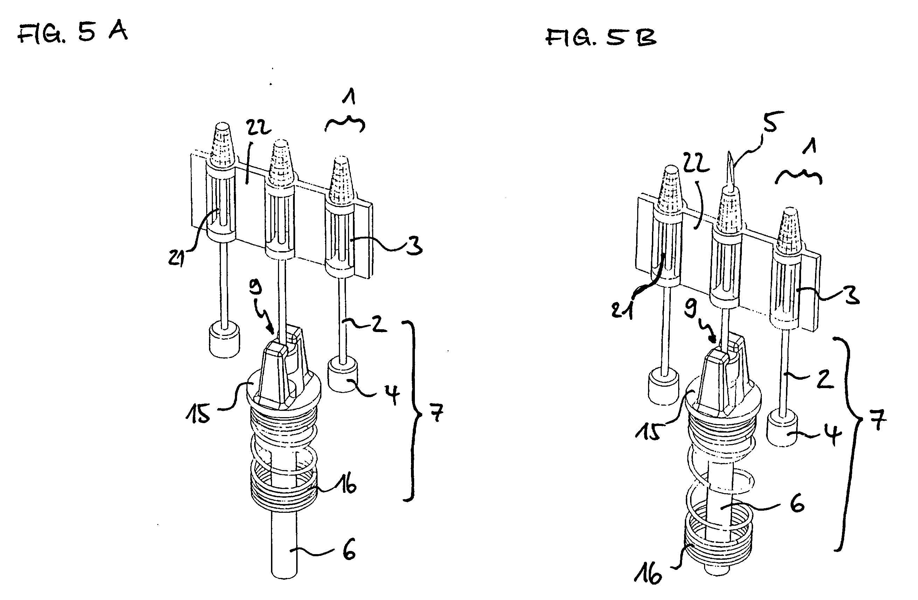

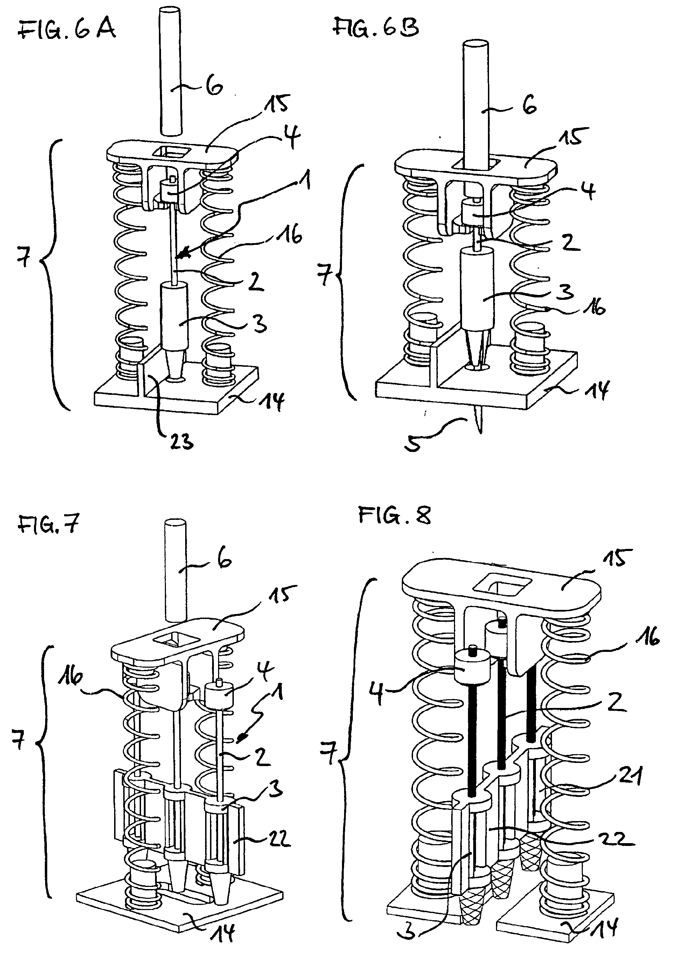

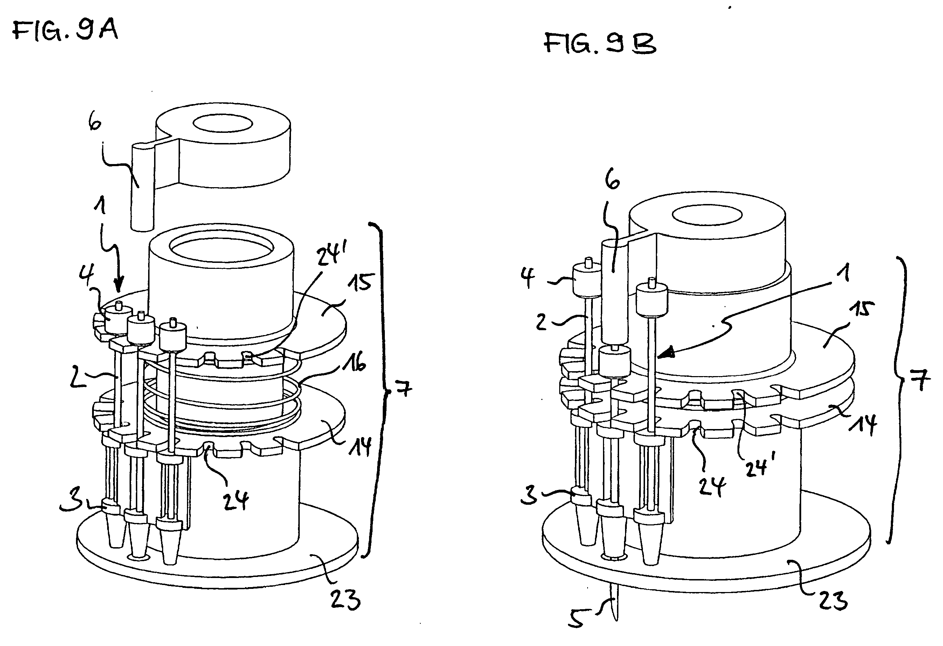

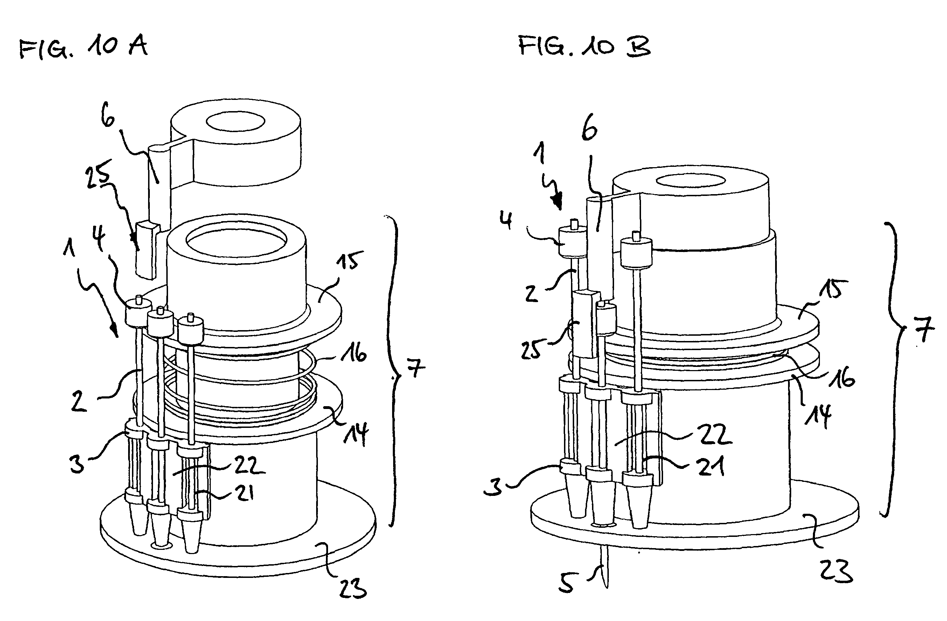

Es versteht sich von selbst, dass Lanzetten, Antriebseinheit und Rückholvorrichtung des erfindungsgemäßen Systems aneinander angepasst sein müssen. Beispielsweise kann die Lanzette mit der Rückholeinrichtung zumindest in Rückwärtsrichtung in Formschluß gebracht werden. Dies kann dadurch geschehen, dass die Lanzette wie ein Kulissenstein bei einer Werkzeugmaschine ausgeformt ist, der in eine entsprechende T-Nut oder einen einfachen Schlitz in einem Blech eingreift (vgl. bspw. Fig. 1A und 1B, weiter unten). Für den Weitertransport der Lanzette kann der Antriebsmechanismus auch ausgerückt werden, wodurch sich die Möglichkeit eröffnet, den Rückholmechanismus als federbelastete Gabel auszuführen, die einfach hinter das verdickte Ende der Lanzette greift (siehe Fig. 3, weiter unten). Der Schlitz in der als Blattfeder ausgeführten Rückholfeder kann aber auch im Einlauf der Lanzetten konisch verengt werden und im Auslauf wieder weiter werden, so daß die Lanzette nur eine glatte Nadel ist, die in der Mittte, der Engstelle, seitlich geklemmt und somit an die Rückholfeder angedockt wird. Der stumpfe Stößel des Antriebs sorgt dann für das Vorfahren der Nadel, die Feder nimmt sie wieder mit zurück, wenn der Stößel sich zurückzieht.

Bevorzugt sind die Lanzetten des erfindungsgemäßen Systems in einem Lanzettenmagazin untergebracht. Dieses kann nach dem Aufbrauchen der Lanzetten aus dem System entnommen und durch ein neues Magazin ersetzt werden. Auf diese Weise kann der Benutzer des Systems auf einfache Weise die aufgebrauchten Lanzetten durch neue ersetzen.

In dem System enthält das Lanzettenmagazin vorteilhafterweise auch die Rückholvorrichtung für die Lanzetten. Insbesondere für den Fall, dass die Rückholvorrichtung einen Federmechanismus aufweist, ist dies ein Vorteil: Mit jedem Magazinwechsel steht dem System eine neue, unverbrauchte Antriebsfeder für den Rückholmechanismus zur Verfügung. Ermüdungserscheinungen des Federmaterials können so weitgehend vermieden werden. Dadurch wird eine gleichbleibende Bewegungsausführung ermöglicht, die wiederum der Schmerzreduktion beim Stechen dient. Zudem ist bei einer solchen Ausführungsform keine Schnittstelle zwischen Rückholvorrichtung und dem Gerät, welches das Lanzettenmagazin aufnimmt, erforderlich. Das Magazin und somit auch das Gesamtsystem enthaltend das Magazin können so sehr kompakt aufgebaut sein.

Das erfindungsgemäße Magazin für Lanzetten enthält neben einer Vielzahl von im wesentlichen nadelförmigen Lanzetten unter anderem einen Aufbewahrungsbereich zum Aufbewahren der Lanzetten und einen Entnahmebereich zum Herausführen zumindest der Spitze einer Lanzette aus dem Magazin während des Stechvorgangs. Das Magazin weist im Entnahmebereich eine Rückholvorrichtung auf, die mit der Lanzette, die sich im Entnahmebereich befindet, wechselwirken kann, um die Lanzette nach der Ausführung des Stechvorgangs von der Stechposition in ihre Ruheposition zu bewegen. Details der einzelnen Komponenten des Lanzettenmagazins entsprechen im wesentlichen den weiter oben im Zusammenhang mit dem erfindungsgemäßen System Gesagten.

Eine bevorzugte Ausführungsform des Lanzettenmagazins enthält zusätzlich eine Transporteinheit, die Lanzetten aus dem Aufbewahrungsbereich in den Entnahmebereich transportieren kann. Die Transportvorrichtung kann vorzugsweise mit einer entsprechenden Antriebseinheit in einem Blutgewinnungsgerät (hier auch kurz als "Stechhilfe" bezeichnet) wechselwirken, wenn sich das Magazin in einem solchen Gerät befindet. In diesem Fall kann das Magazin einen Teil des Antriebsgetriebes für die Transporteinheit enthalten, während im Blutgewinnungsgerät beispielsweise ein Antriebsmotor für die Transporteinheit sitzt. Es ist jedoch auch möglich, dass die Transporteinheit, insbesondere ihr Antriebsmotor und ihr Getriebe, vollständig im Magazin sitzt und nur ihre Steuerung und gegebenenfalls Energieversorgung von außerhalb des Magazins vorgenommen wird.

Ein weiterer Gegenstand der Erfindung ist eine Stechhilfe , die zum Einsatz in einem System zum Bevorraten und Bereitstellen von Lanzetten zur Gewinnung von Körperflüssigkeiten, insbesondere zur Blutgewinnung, wie es oben beschrieben wurde, geeignet ist. Wie das erfindungsgemäße System umfasst die Stechhilfe eine Antriebseinheit, die ein Antriebselement (beispielsweise einen Hammer oder einen Stößel) besitzt, das zur Ausführung eines Stechvorgangs aus einer Ruheposition in eine Stechposition bewegt wird. Ferner weist die Stechhilfe einen Aufbewahrungsbereich zum Aufbewahren von Lanzetten und einen Entnahmebereich zum Herausführen zumindest der Spitze einer Lanzette aus dem System während des Stechvorgangs auf. Zusätzlich enthält die Stechhilfe eine Transporteinheit, die Lanzetten aus dem Aufbewahrungsbereich in den Entnahmebereich transportieren kann. Das Antriebselement ist so angeordnet, dass es im Entnahmebereich auf die dort befindliche Lanzette zur Ausführung des Stechvorgangs einwirken kann, und diese so aus einer Ruheposition in eine Stechposition bewegt wird. Die Stechhilfe weist in einer Ausführungsform im Entnahmebereich eine Rückholvorrichtung auf, die mit der Lanzette, die sich im Entnahmebereich befindet, wechselwirken kann, um die Lanzette nach der Ausführung des Stechvorgangs von der Stechposition in ihre Ruheposition zu bewegen. Die einzelnen Elemente der Stechhilfe entsprechen dabei im Wesentlichen den oben beschriebenen Elementen des erfindungsgemäßen Systems.

Das beispielhafte Verfahren dient zum zeitweisen Ausfahren der Spitze einer im wesentlichen nadelförmigen Lanzette aus einem System zum Bevorraten und Bereitstellen von Lanzetten zur Gewinnung von Körperflüssigkeiten, insbesondere zur Blutgewinnung. Es eignet sich insbesondere für das erfindungsgemäße, oben beschriebene System. Bei dem Verfahren wird ein Antriebselement, beispielsweise ein Stößel, aus seiner Ruheposition in Richtung seiner Stechposition bewegt. Dabei treibt es eine Lanzette an, bis diese aus ihrer Ruheposition ihre Stechposition erreicht hat. Nachdem die Lanzette ihre Stechposition erreicht hat, wird sie mit Hilfe einer Rückholvorrichtung in ihre Ruheposition zurückgeführt. Die Vorwärtsbewegung der Lanzette, das heißt ihre Bewegung aus der Ruheposition in die Stechposition, wird so von der Antriebseinheit, insbesondere dem Antriebselement bewirkt; die Rückwärtsbewegung wird durch die von der Antriebseinheit unabhängige, separate Rückholvorrichtung bewirkt. Die Rückholvorrichtung kann dabei neben der Lanzette auch das Antriebselement in seine Ruheposition zurückführen.

Die Erfindung wird durch die nachfolgenden Figuren 1 bis 11 näher erläutert.

Figur 1 zeigt für eine bevorzugte Ausführungsform schematisch einen Ausschnitt aus dem erfindungsgemäßen System, in dem das Wechselwirken von Antriebselement, Lanzette und Rückholvorrichtung vor der Stechbewegung (Fig. 1 A) und am Ende der Stechbewegung (Fig. 1 B) dargestellt ist.

Figur 2 zeigt für eine bevorzugte Ausführungsform schematisch eine perspektivische, teilweise aufgeschnittene Ansicht in ein Lanzettenmagazin des erfindungsgemäßen Systems (Fig. 2 A) sowie eine entsprechende Aufsicht, in der die Abdeckung des Magazingehäuses weggelassen wurde (Fig. 2 B).

Figur 3 zeigt schematisch eine weitere, alternative Ausführungsform des erfindungsgemäßen Systems.

Figur 4 zeigt für eine weitere bevorzugte Ausführungsform schematisch einen Ausschnitt aus dem erfindungsgemäßen System, in dem das Wechselwirken von Antriebselement, Lanzette und Rückholvorrichtung vor der Stechbewegung (Fig. 4 A) und am Ende der Stechbewegung (Fig. 4 B) dargestellt ist.

Figur 5 zeigt für eine weitere bevorzugte Ausführungsform schematisch einen Ausschnitt aus dem erfindungsgemäßen System, in dem das Wechselwirken von Antriebselement, Lanzette und Rückholvorrichtung vor der Stechbewegung (Fig. 5 A) und am Ende der Stechbewegung (Fig. 5 B) dargestellt ist.

Figur 6 zeigt für eine weitere bevorzugte Ausführungsform schematisch einen Ausschnitt aus dem erfindungsgemäßen System, in dem das Wechselwirken von Antriebselement, Lanzette und Rückholvorrichtung vor der Stechbewegung (Fig. 6 A) und am Ende der Stechbewegung (Fig. 6 B) dargestellt ist.

Figur 7 zeigt schematisch eine Variante des Systems aus Figur 6, wobei hier nur die Situation vor der Stechbewegung abgebildet ist.

Figur 8 zeigt eine weitere Variante des Systems aus Figur 6, wobei hier ebenfalls nur die Situation vor der Stechbewegung abgebildet ist.

Figur 9 zeigt für eine weitere bevorzugte Ausführungsform schematisch einen Ausschnitt aus dem erfindungsgemäßen System, in dem das Wechselwirken von Antriebselement, Lanzette und Rückholvorrichtung vor der Stechbewegung (Fig. 9 A) und am Ende der Stechbewegung (Fig. 9 B) dargestellt ist.

Figur 10 zeigt für eine weitere bevorzugte Ausführungsform, die als Variante zu der in Figur 9 dargestellten Auführungsform anzusehen ist, schematisch einen Ausschnitt aus dem erfindungsgemäßen System, in dem das Wechselwirken von Antriebselement, Lanzette und Rückholvorrichtung vor der Stechbewegung (Fig. 10 A) und am Ende der Stechbewegung (Fig. 10 B) dargestellt ist.

Figur 11 zeigt für eine weitere bevorzugte Ausführungsform, die als weitere Variante zu der in Figur 9 dargestellten Auführungsform anzusehen ist, schematisch einen Ausschnitt aus dem erfindungsgemäßen System, in dem das Wechselwirken von Antriebselement, Lanzette und Rückholvorrichtung vor der Stechbewegung (Fig. 11 A) und am Ende der Stechbewegung (Fig. 11 B) dargestellt ist.

Die Ziffern und Buchstaben in den Figuren bedeuten:
- 1: Lanzette
- 2: Lanzettennadel
- 3: Lanzettenkörper
- 4: Lanzettenkopf
- 5: Lanzettennadelspitze
- 6: Antriebselement
- 7: Rückholvorrichtung
- 8: Blattfeder
- 9: Schlitz
- 10: Lanzettenmagazin
- 11: Gehäuse
- 12: Führungskanal
- 13: Öffnung
- 14: feststehendes Teil der Rückholvorrichtung 7
- 15: bewegliches Teil der Rückholvorrichtung 7
- 16: Schraubenfeder
- 17: Schubgestänge
- 18: Transportvorrichtung
- 19: Kippachse
- 20: Nocken
- 21: Aussparung im Lanzettenkörper 3
- 22: Verbindungsstege der Lanzettenkörper 3
- 23: Anschlag für Lanzettenkörper 3
- 24: Aussparung
- 25: Führungselement des Anstriebselements 6
- 26: Führungselement
- A: Bewegungsrichtung des Antriebselements 6
- B: Bewegungsrichtung des Schubgestänges 17
- C: Bewegungsrichtung der Rückholvorrichtung 7 beim Kippen um Achse 19
- D: Bewegungsrichtung der Transportvorrichtung 18

In Figur 1 ist schematisch ein Ausschnitt aus einer bevorzugten Ausführungsform des erfindungsgemäßen Systems dargestellt. Figur 1 dient im Wesentlichen dazu, die Wechselwirkung zwischen der Lanzette (1), dem Antriebselement (6) der Antriebseinheit, und der Rückholvorrichtung (7) zu erläutern. Von den fünf in Figur 1 dargestellten Lanzetten (1) befindet sich die zweite Lanzette von links direkt über dem Antriebselement (6). Diese Position markiert den Entnahmebereich des erfindungsgemäßen Systems. Die dort befindliche Lanzette (1) kann in dieser Position vom Antriebselement (6) zum Ausführen der Stechbewegung angetrieben werden. Gleichzeitig liegt die Lanzette (1), die sich im Entnahmebereich befindet innerhalb der schlitzförmigen Aussparung (9) der Blattfeder (8), welche Teil der Rückholvorrichtung (7) ist.

Die Lanzette (1) enthält neben der Lanzettennadel (2) einen Lanzettenkörper (3), der vor der Benutzung die Spitze (5) der Lanzettennadel (2) umhüllt. An dem der Spitze (5) abgewandten Ende der Lanzettennadel (2) enthält die Lanzette (1) einen Lanzettenkopf (4). Dieser und der Lanzettenkörper (3) können im Spritzgussverfahren um die Lanzettennadel (2) gespritzt sein und bestehen vorzugsweise aus einem spritzgussfähigen Kunststoff. Vorzugsweise besteht der Lanzettenkörper (3) aus einem elastischen Material, während der Lanzettenkopf (4) aus einem starren Material besteht. Es ist jedoch möglich, beide Bestandteile der Lanzette (1) aus einem starren Material zu formen.

In Figur 1A befindet sich die Lanzette (1'), die sich im Entnahmebereich befindet, in ihrer Ruheposition. Figur 1A repräsentiert somit den Zustand des Systems vor oder nach Ausführung einer Stechbewegung.

In Figur 1B befindet sich die Lanzette (1'), die sich im Entnahmebereich befindet, in der Stechposition. Dabei ist die Nadelspitze (5) aus dem Lanzettenkörper (3) ausgefahren. Das Antriebselement (6) wirkt dabei auf das der Lanzettennadelspitze (5) gegenüber liegende Ende der Lanzettennadel (2) ein. Der Lanzettenkopf (4) wechselwirkt mit der Blattfeder (8) der Rückholvorrichtung (7) und dehnt diese aus ihrer Ruhelage heraus. Der Schlitz (9) der Blattfeder (8) ist breit genug, um die Lanzettennadel (2) aufnehmen zu können. Andererseits ist er schmal genug, um eine Angriffsfläche für den Lanzettenkopf (4) zu bieten.

Sobald vom Antriebselement (6) keine Kraft auf die Lanzette (1') mehr ausgeübt wird, sorgt die Blattfeder (8) der Rückholvorrichtung (7) dafür, dass die Lanzette (1'), die sich im Entnahmebereich befindet, in ihre Ruheposition zurückgeführt wird.

Während der Stechbewegung wird der Lanzettenkörper (3) in seiner Position fixiert. Dies kann entweder dadurch geschehen, dass die einzelnen Lanzettenkörper (3) der unterschiedlichen Lanzetten (1) direkt miteinander verbunden sind, beispielsweise über gemeinsame Verbindungsstege, oder aber dadurch, dass die Lanzettenkörper (3) an einem steifen, bandförmigen Material, welches hier in den Figuren nicht gezeigt ist, fixiert sind. Alternativ kann die Fixierung durch das Magazin bzw. dessen Gehäuse bewirkt werden.

Nach dem Ausführen der Stechbewegung und dem Zurückführen der Lanzettennadel (2) in ihre Ruheposition wird die benutzte Lanzette (1') aus dem Entnahmebereich in den Aufbewahrungsbereich transportiert. Dabei wird gleichzeitig die nächste Lanzette (1") aus dem Aufbewahrungsbereich in den Entnahmebereich bewegt.

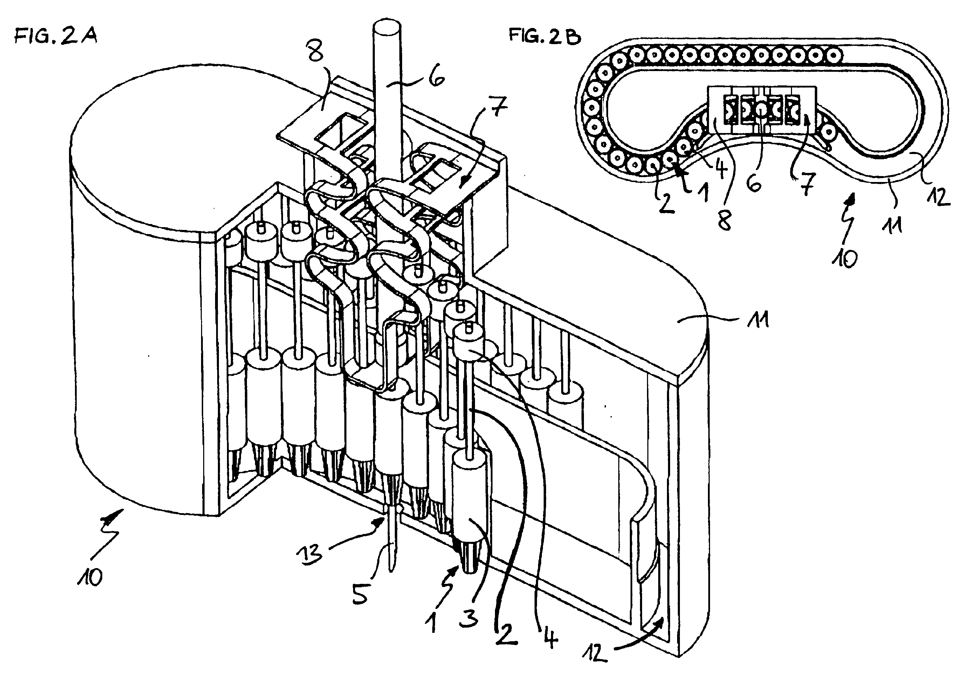

In Figur 2 ist eine mögliche Ausführungsform für die in Figur 1 beschriebenen Bestandteile des erfindungsgemäßen Systems abgebildet. Figur 2A zeigt in einer perspektivischen, teilweise aufgeschnittenen Ansicht ein Lanzettenmagazin (10), in welches die in Figur 1 gezeigten Elemente des erfindungsgemäßen Systems integriert sind. Figur 2B zeigt dasselbe Magazin (10) in einer schematischen Aufsicht, wobei der obere Deckel des Lanzettenmagazingehäuses (11) weggelassen wurde, um einen Einblick in das Innere eines Magazins (10) zu gewähren.

In Figur 2A ist das System in dem bereits in Figur 1B dargestellten Zustand wiedergegeben. Das Antriebselement (6) der Antriebseinheit drückt dabei von oben auf die Lanzettennadel (2) der Lanzette (1), welche sich im Entnahmebereich befindet. Die Lanzettennadelspitze (5) ist aus dem Lanzettenkörper (3) hinausgeschoben und die Blattfeder (8) der Rückholvorrichtung (7) befindet sich im gedehnten Zustand. Das Magazin (10) weist in seinem Gehäuse (11) eine Öffnung (13) auf, aus der die Lanzettennadelspitze (5) zur Ausführung des Stechvorgangs austreten kann.

Wie insbesondere aus Figur 2B ersichtlich ist, werden die miteinander verbundenen Lanzetten (1) in einem Führungskanal (12) des Gehäuses (11) des Magazins (10) vom Aufbewahrungsbereich in den Entnahmebereich und nach ausgeführter Stechbewegung zurück in den Aufbewahrungsbereich bewegt. Die einzelnen Lanzetten (1) sind sowohl vor als auch nach Ausführung des Stechvorgangs sicher durch das Gehäuse (11) des Magazins (10) von der Umgebung abgeschirmt.

Da die Lanzetten (1) weitgehend miniaturisierbar sind, kann auf geringstem Raum eine große Anzahl von ihnen untergebracht werden. Durch die Integration der Rückholvorrichtung (7) in das Lanzettenmagazin (10) wird gewährleistet, dass die Blattfeder (8) der Rückholvorrichtung (7) mit jedem Magazinwechsel erneuert wird und somit ein Ermüden des Materials der Rückholvorrichtung (7) ausgeschlossen wird. Das Magazin macht zudem das Ankoppeln von Stechhilfe und Lanzetten einfach und sicher.

In Figur 3 ist schematisch eine weitere alternative Ausführungsform des erfindungsgemäßen Systems gezeigt. Im Wesentlichen sind in Figur 3 eine Vielzahl von im Wesentlichen nadelförmigen Lanzetten (1), eine Antriebsvorrichtung mit dem Antriebselement (6), eine Rückholvorrichtung (7) und eine Transportvorrichtung (18) zu sehen.

Die einzelnen Lanzetten (1) sind im Bereich ihres Lanzettenkörpers (3) miteinander verbunden. Im gezeigten Fall sind die Lanzettenkörper (3) auf ein Transportband aufgebracht. Es ist jedoch auch möglich, dass die einzelnen Lanzettenkörper (3) über sich jeweils berührende Kanten miteinander über Gelenke oder Filmscharniere verbunden sind.

Wie schon die Lanzetten aus Figur 1 und Figur 2 besitzen die Lanzetten (1) aus Figur 3 eine Lanzettennadel (2) mit einer Lanzettennadelspitze (5), die im Ruhezustand im Lanzettenkörper (3) eingeschlossen ist. Zur Sicherung der Sterilität der Lanzettennadelspitze (5) vor Gebrauch, kann der Lanzettenkörper (3) oben mit einem elastischen von der Lanzettennadelspitze (5) durchstoßbaren Material verschlossen sein (nicht gezeigt). An der der Spitze abgewandten Seite der Lanzettennadel (2) befindet sich der Lanzettenkopf (4). Der Lanzettenkopf (4) und der Lanzettenkörper (3) sind vorzugsweise aus einem spritzgussfähigen Material an die Lanzettennadel (2) angespritzt. Sie können aus denselben oder aus unterschiedlichen Materialien bestehen.

Eine der in Figur 3 gezeigten Lanzetten (1) befindet sich direkt über dem Antriebselement (6) und somit im Entnahmebereich. Die übrigen Lanzetten befinden sich im Aufbewahrungsbereich.

Im Entnahmebereich greift die Rückholvorrichtung (7) an die dort befindliche Lanzette (1') an. Im vorliegenden Fall enthält die Rückholvorrichtung (7) ein im Wesentlichen feststehendes Teil (14) und ein bewegliches Teil (15). Beide Teile weisen eine im Wesentlichen halbkreisförmige Aussparung auf, die die Lanzettennadel (2) der Lanzette (1') im Entnahmebereich teilweise umfassen können.

Wird das Antriebselement (6) entlang der Richtung A nach oben bewegt, um die Lanzette (1'), die sich in der Entnahmeposition befindet, zum Stechen aus ihrer Ruheposition auszulenken, so trifft der Lanzettenkopf (4) auf den beweglichen Teil (15) der Rückholvorrichtung (7). Bei der Ausführung der Stechbewegung wird der bewegliche Teil (15) gegen den feststehenden Teil (14) verschoben und dadurch die Schraubenfeder (16) zusammengedrückt. Nach dem Ausführen der Stechbewegung kehrt das Antriebselement (6) in Richtung A nach unten in seine Ausgangsposition zurück. Die zusammengedrückte Schraubenfeder (16) der Rückholvorrichtung (7) kann sich nunmehr entspannen und drückt dabei den beweglichen Teil (15) nach unten. Dabei drückt der bewegliche Teil (15) von oben auf den Lanzettenkopf (4) und führt somit die Lanzette (1') aus der Stechposition in ihre Ruheposition.

Danach kann die benutzte Lanzette (1') aus dem Entnahmebereich in den Aufbewahrungsbereich transportiert werden. Gleichzeitig wird eine weitere Lanzette - im vorliegenden Fall, die sich rechts neben der benutzten Lanzette (1') befindlichen Lanzette (1") - aus dem Aufbewahrungsbereich in den Entnahmebereich transportiert. Dazu wird das Schubgestänge (17) in die Richtung B bewegt. Diese Bewegung führt dazu, dass einerseits die Rückholvorrichtung (7) um die Achse (19) in Richtung C gekippt wird (Dieser Zustand ist in gestrichelten Linien angedeutet). Durch diese Kippbewegung wird die Lanzette (1'), die sich im Entnahmebereich befindet, von der Rückholvorrichtung (7) freigegeben, d. h. die Teile 14 und 15 werden von der Lanzettennadel (2) zurückgezogen. Gleichzeitig bewirkt die Bewegung des Schubgestänges (17) in Richtung B ein Drehen der Transportvorrichtung (18) in Richtung D. Die Übertragung der Bewegung des Schubgestänges (17) auf die Transportvorrichtung (18) kann beispielsweise über einen angeschrägten Nocken (20) erfolgen. Durch die Bewegung der Transportvorrichtung in Richtung D wird die nächste Lanzette (1") aus dem Aufbewahrungsbereich in den Entnahmebereich befördert und gleichzeitig die benutzte Lanzette (1') aus dem Entnahmebereich in den Aufbewahrungsbereich überführt. Durch Zurückbewegen des Schubgestänges (17) in seine Ausgangsposition wird die Rückholvorrichtung (7) ebenfalls zurückgekippt und kann somit wieder an die nächste zu benutzende Lanzette (1) angreifen.

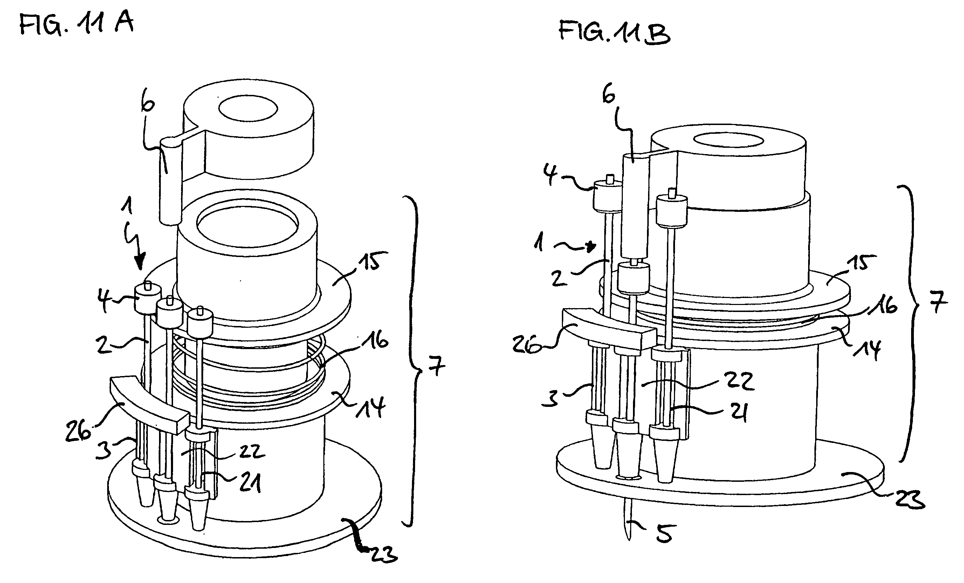

In Figur 4 ist eine Variante des erfindungsgemäßen Systems aus Figur 1 schematisch dargestellt. In dieser Variante ist die Blattfeder (8) der Rückholvorrichtung (7) anders geformt, wobei diese Formgebung bei einfacherer Ausführung ähnliche Funktionalität wie die in Figur 1 gezeigte Form aufweist. Die Teilfigur 4 A zeigt die Verhältnisse vor bzw. nach dem Stechvorgang; Teilfigur 4 B zeigt die Verhältnisse mit der Lanzette (1) in der Stechposition.

Die einzelnen Lanzetten (1) sind hier - im Vergleich zu der Ausführungsform der Figur 1 - wieter voneinander entfernt. Die Lanzettenkörper (3) sind über Verbindungsstege (22) miteinander verbunden, die ein Fixieren der Lanzetten (1) während des Stechvorgangs bewirken bzw. unterstützen können.

Die Lanzette (1) in dieser Ausführungsform enthält einen Lanzettenkörper (3), welcher die Lanzettennadel (2) im Bereich ihres Schaftes an zwei Stellen umschließt. In alternativen Ausführungsformen können natürlich auch mehrere Stellen der Lanzettennadel (2) umschlossen sein. Das stumpfe Ende der Nadel (2) kann einen angespritzten Lanzettenkopf (4) oder ein äquivalentes Element aufweisen, welches zur Kopplung an die Rückholvorrichtung (7) dient. Die Nadelspitze (5) ist in den Kunststoff des Lanzettenkörpers (3) eingebettet, um die Sterilität der Nadelspitze (5) bis zum Gebrauch sicherzustellen. Alternativ kann die Lanzettennadelspitze (5) in einem Hohlraum, welcher auf einer Seite mit einer Folie zugeschweißt ist, verschlossen aufbewahrt werden (vgl. Figur 3).

Die Lanzette (1) ist so aufgebaut, dass bei fixiertem Lanzettenkörper (3) (durch Lanzettenmagazin (10) oder Gerät) die Lanzettennadel (2), im Lanzettenkörper(3), in Stechrichtung bewegt werden kann. Um das Herausschieben der Lanzettennadel(2) aus dem Kunststoff des Lanzettenkörpers (3) zu erleichtern, ist der Nadelschaft an den umspritzten Stellen möglichst glatt gehalten, beispielsweise poliert. Weiterhin kann die Lanzettennadel (2) im Bereich der Umspritzung mit einem geeigneten Gleitmittel versehen sein, um ein besseres Gleiten der Lanzettennadel durch den Kunststoff des Lanzettenkörpers (3) zu ermöglichen.

Durch das Umschließen des Schaftes der Lanzettennadel (2) an mindestens zwei Stellen längs des Nadelschaftes, kann die Lanzettennadel (2) axial exakt positioniert werden. Die Umschließung des Nadelschaftes ist so ausgeführt, dass minimale Oberflächen der Lanzettennadel (2) mit Kunststoff des Lanzettenkörpers (3) umspritzt sind, um möglichst geringe Reibungswiderstände (möglichst kleiner als 1 N) zu erreichen. Der gewählte Kunststoff sollte so formstabil sein, dass er sich nicht beim Ausführen der Stechbewegung verformt. Als geeignet haben sich beispielsweise Polyethylen oder Polypropylen erwiesen. Durch die Formstabilität des Lanzettenkörpers (3) und die Einbettung der Lanzettennadel (2) an mindestens zwei Punkten wird erreicht, dass die Lanzettennadel (2) bei fixiertem Lanzettenkörper (3) weitgehend spielfrei geführt wird und nicht seitlich schwingen kann. Dadurch kann der Schmerz beim Stechen reduziert werden.

Das Antriebselement (6) bewegt die Lanzettennadel (2) in Stechrichtung durch den Lanzettenkörper (3). Dabei wird die Lanzettennadel (2) durch das Antriebselement (6) seitlich nicht geführt. Die seitliche Führung wird durch den Lanzettenkörper (3) bzw. den Verbund der einzelnen Lanzettenkörper (3) bewirkt.

Für die in Figur 4 gezeigte Lanzettenform hat es sich als vorteilhaft herausgestellt, für den Lanzettenkörper (3), der gleichzeitig dem Sterilschutz dient, ein so weiches Material zu wählen (mit einer Shore Härte A von 30 und weniger), dass die Lanzettennadelspitze (5) aufgrund ihres asymmetrischen Schliffs bei der Stechbewegung nicht zu stark seitlich ausweicht und die Reibungskräfte beim Stechen möglichst gering sind (vorzugsweise kleiner als 1 N).

Beim Umspritzen der Lanzettennadelspitze (5) mit dem Kunststoff des Lanzettenkörpers (3) ist die Geometrie (Form) und die Wandstärke der Umspritzung der Lanzettennadelspitze (5) so zu gestalten, dass die Lanzettennadelspitze (5) den Kunststoff leicht durchdringen kann und die Reibungskräfte beim Stechen möglichst gering sind (möglichst kleiner als 2 N).

In Figur 5 ist eine weitere, zu der Ausführungsform der Figuren 1 und 4 alternative Ausführungsform des erfindungsgemäßen Systems schematisch dargestellt. Die Teilfigur 5 A zeigt die Verhältnisse vor bzw. nach dem Stechvorgang; Teilfigur 5 B zeigt die Verhältnisse mit der Lanzette (1) in der Stechposition.

Die Lanzette (1) bzw. der Lanzettenverband entspricht in Figur 5 im Wesentlichen der Ausführungsform der Figur 4. Die Rückholvorrichtung (7) enthält in der hier gezeigten Ausführungsform ein bewegliches Teil (15), das eine Greifvorrichtung mit einem Schlitz (9) zur Aufnahme der Lanzette (1) aufweist. Das bewegliche Teil (15) ist über eine Zugschraubenfeder (16) mit einem feststehenden Teil (nicht gezeigt) der Rückholvorrichtung (7) verbunden. Während der Phase der Stechbewegung, während der das Antriebselement (6) auf die Lanzette (1) einwirkt, wird die Feder (16) gedehnt, da die Lanzette (1) über ihren Kopf (4) mit dem beweglichen Teil (15) der Rückholvorrichtung verbunden ist. Sobald die Kraftübertragung durch das Antriebselement (6) beendet ist - das heißt, sobald die Lanzettennadelspitze (5) zum Stechen vollständig ausgefahren ist - kann die Zugfeder (16) in ihre entspannte Ausgangslage zurückkehren. Sie nimmt dabei die Lanzettennadel (2) mit in die Ausgangsposition zurück und befördert gegebenenfalls auch das Antriebselement (6) in die Ausgangslage.

In Figur 6 ist ebenfalls eine alternative Ausführungsform des erfindungsgemäßen Systems schematisch abgebildet. Die Lanzette (1) entspricht dabei im Wesentlichen der Ausführungsform der Figur 1. Die Teilfigur 6 A zeigt die Verhältnisse vor bzw. nach dem Stechvorgang; Teilfigur 6 B zeigt die Verhältnisse mit der Lanzette (1) in der Stechposition, das heißt mit ausgefahrener Lanzettennadelspitze (5).

Wie in Figur 5 wird hier die Rückholvorrichtung aus zwei gegeneinander beweglichen Teilen (14, 15) gebildet, von denen eines (14) feststehend ist und eines (15) bei der Stechbewegung mit der Lanzette (1) zusammenwirkt, indem sie die Lanzette (1) an ihrem Kopf (4) umfaßt, und mit dieser beweglich ist. Das feststehende Teil (14) enthält eine Aussparung (Loch) für den Durchtritt der Lanzettennadelspitze (5). Anders als in Figur 5 wird in Figur 6 die Rückholvorrichtung (7) durch eine Druckschraubenfeder (16) ergänzt, die beim Ausführen der Stechbewegung zusammengedrückt wird. Sobald die Kraftübertragung durch das Antriebselement (6) beendet ist - das heißt, sobald die Lanzettennadelspitze (5) zum Stechen vollständig ausgefahren ist - kann die Druckfeder (16) in ihre entspannte Ausgangslage zurückkehren. Sie nimmt dabei die Lanzettennadel (2) mit in die Ausgangsposition zurück und befördert gegebenenfalls auch das Antriebselement (6) in die Ausgangslage.

Die in Figur 6 dargestellte Ausführungsform enthält auf der der Lanzette (1) zugewandten Seite des feststehenden Teils (14) der Rückholvorrichtung (7) einen Anschlag (23) für den Lanzettenkörper (3), der verhindert, dass sich der Lanzettenkörper (3) bei der Stechbewegung mit der Lanzettennadel (2) zusammen bewegt. Dadurch ist gewährleistet, dass sich die Lanzettennadel (2) mit ihrer Spitze (5) aus dem Körper (3) herausbewegen kann und so für den Stechvorgang zur Verfügung steht.

Die in den Figuren 7 und 8 abgebildeten Ausführungsformen des erfindungsgemäßen Systems sind Varianten der Ausführungsform der Figur 6. Sowohl Figur 7 als auch Figur 8 zeigen nur die Ausgangsposition des Systems. In beiden entsprechen die Lanzetten (1) bzw. die entsprechenden Lanzettenverbände aus mehreren Lanzetten denjenigen aus Figur 4 und 5. Da die Lanzetten (1) hier über Stege (22) miteinander verbunden sind, die eine relative Fixierung der Lanzettenkörper (3) gegeneinander bewirken, ist ein Anschlag (23) entbehrlich.

Die feststehenden Teile (14) der Rückholvorrichtung (7) weisen in den Figuren 7 und 8 bezüglich der Ausführungsform von Figur 6 alternative Aussparungen auf (länglicher Schlitz in Figur 7; über einen Spalt getrennte Teile in Figur 8).

In Figur 9 ist eine weitere alternative Ausführungsform des erfindungsgemäßen Systems schematisch dargestellt (Figur 9 A: vor bzw. nach Ausführen der Stechbewegung; Figur 9 B während des Stechvorgangs). Die Lanzetten (1) werden hier im wesentlichen kreisförmig um die Rückholvorrichtung (7) bewegt. Die Lanzetten (1) bzw. die entsprechenden Lanzettenverbände aus mehreren Lanzetten entsprechen im Wesentlichen denjenigen aus Figur 4 und 5. Das Antriebsprinzip entspricht demjenigen aus den Figuren 6 bis 8: die Lanzetten (1) werden von einem Antriebselement (6) aus ihrer Ruhepostion in die Stechposition bewegt. Die Lanzette (1) wechselwirkt dabei über ihren Kopf (4) mit dem beweglichen Teil (15) der Rückholvorrichtung (7). Das bewegliche Teil (15) bewegt sich dabei auf das feststehende Teil (14) zu und drückt eine Schraubenfeder (16) zusammen. Die Lanzetten (1) werden hierbei im Bereich der freiliegenden Nadel (2) durch Aussparungen (24) im feststehenden und beweglichen Teil (14, 15) der Rückholvorrichtung (7), die hier die Form eines Zahnkranzes aufweisen, seitlich fixiert und so geführt. Die Rückholvorrichtung (7) weist einen miteiner Aussparung versehenen Kranz auf, der als Anschlag (23) für die Lanzettenkörper (3) dient und dafür sorgt, dass die Lanzettenkörper (3) sich bei der Stechbewegung nicht zusammen mit der Lanzettennadel (2) bewegen.

Die zahnkranzförmigen Teile (14, 15) der Rückholvorrichtung (7) dieser Ausführungsform können auch dem Weitertransport der Lanzetten dienen.

Figur 10 zeigt in ebenfalls zwei Teilfiguren (Figur 10 A: vor bzw. nach Ausführen der Stechbewegung; Figur 10 B während des Stechvorgangs) eine Variante der Ausführungsform gemäß Figur 9. Im Unterschied zur dort gezeigten Ausführungsform weisen die Teile (14, 15) der Rückholvorrichtung (7) keine Aussparungen auf. Die Führung der Lanzettennadel (2) während des Stechvorgangs wird hier durch das Führungselement (25) des Antriebselements (6) bewerkstelligt.

Figur 11 zeigt in ebenfalls zwei Teilfiguren (Figur 11 A: vor bzw. nach Ausführen der Stechbewegung; Figur 11 B während des Stechvorgangs) eine Variante der Ausführungsform gemäß Figur 10. Im Unterschied zur dort gezeigten Ausführungsform wird die Führung der Lanzettennadel (2) hier über ein Führungselement (26), welches beispielsweise an einer Gehäusewand eines entsprechenden Lanzettenmagazins befestigt sein kann, bewirkt.

Selbstverständlich können in allen gezeigten Ausführungsformen mehr oder weniger als die gezeigten Lanzetten (1) enthalten sein. Auch ist es möglich, die unterschiedlichen Lanzettenformen in den gezeigten Beispielen gegeneinander auszutauschen oder andere als die gezeigten Lanzetten (1) einzusetzen.

## Patentansprüche

1. System zum Bevorraten und Bereitstellen von Lanzetten zur Gewinnung von Körperflüssigkeiten, umfassend
i) eine Vielzahl von im Wesentlichen nadelförmigen Lanzetten (1)
ii) eine Antriebseinheit, die ein Antriebselement (6) besitzt, das zur Ausführung eines Stechvorgangs aus einer Ruheposition in eine Stechposition bewegt wird;
iii) einen Aufbewahrungsbereich zum Aufbewahren der Lanzetten (1);
iv) einen Entnahmebereich zum Herausführen zumindest der Spitze (5) einer Lanzette (1) aus dem System während des Stechvorgangs; und
v) eine Transporteinheit (18), die Lanzetten (1) aus dem Aufbewahrungsbereich in den Entnahmebereich transportieren kann,
wobei das Antriebselement (6) so angeordnet ist, dass es im Entnahmebereich auf die dort befindliche Lanzette (1) zur Ausführung des Stechvorgangs einwirkt und diese so aus einer Ruheposition in eine Stechposition bewegt wird, und
wobei das System im Entnahmebereich eine Rückholvorrichtung (7) aufweist, die mit der Lanzette (1), die sich im Entnahmebereich befindet während die Lanzette (1) zur Ausführung des Stechvorgangs von der Stechposition in ihre Ruheposition bewegt wird, wechselwirkt, wobei die Antriebseinheit und die Rückholvorrichtung (7) unabhängig voneinander an der Lanzette (1) angreifen, **dadurch gekennzeichnet, dass** die Rückführung der Lanzette (1) durch eine zentrale Rückholvorrichtung (7) bewirkt wird, so dass die Rückführung verschiedener Lanzetten durch nur eine Rückholvorrichtung erfolgt.

2. System gemäß Anspruch 1, wobei die Lanzette an ihrem der Spitze gegenüberliegenden Ende ein durch die Rückholvorrichtung greifbares Element aufweist.

3. System gemäß Anspruch 1, wobei die Rückholvorrichtung einen Federmechanismus umfasst.

4. System gemäß einem der vorhergehenden Ansprüche, wobei die Lanzetten in einem Magazin untergebracht sind.

5. System gemäß Anspruch 4, wobei die Rückholvorrichtung in dem Magazin enthalten ist.

6. Lanzettenmagazin (10) zum Bevorraten von Lanzetten (1), umfassend
i) eine Vielzahl von im Wesentlichen nadelförmigen Lanzetten (1)
ii) einen Aufbewahrungsbereich zum Aufbewahren der Lanzetten (1); und
iii) einen Entnahmebereich zum Herausführen zumindest der Spitze (5) einer Lanzette aus dem Magazin (10) während des Stechvorgangs die, mittels einer Antriebseinheit herausführbar ist;
wobei das Magazin (10) im Entnahmebereich eine zentrale Rückholvorrichtung (7) aufweist, die mit der Lanzette (1), die sich im Entnahmebereich befindet, in der Weise wechselwirkt, dass die Rückholvorrichtung (7) unabhängig von einer Antriebseinheit an der Lanzette (1) angreift, um die Lanzette (1) nach der Ausführung des Stechvorgangs von der Stechposition in ihre Ruheposition zu bewegen, **dadurch gekennzeichnet, dass** die Rückführung verschiedener Lanzetten durch nur eine Rückholvorrichtung erfolgt.

7. Lanzettenmagazin gemäß Anspruch 6, enthaltend zusätzlich
iv) eine Transporteinheit, die Lanzetten aus dem Aufbewahrungsbereich in den Entnahmebereich transportieren kann.

8. Lanzettenmagazin gemäß Anspruch 6 oder 7, wobei die Lanzette an ihrem der Spitze gegenüberliegenden Ende ein durch die Rückholvorrichtung greifbares Element aufweist.

9. Lanzettenmagazin gemäß Anspruch 6, 7 oder 8, wobei die Rückholvorrichtung einen Federmechanismus umfasst.

10. Stechhilfe geeignet zum Einsatz in einem System zum Bevorraten und Bereitstellen von Lanzetten (1), in einem Magazin umfassend
i) eine Antriebseinheit, die ein Antriebselement (6) besitzt, das zur Ausführung eines Stechvorgangs aus einer Ruheposition in eine Stechposition bewegt wird;
ii) einen Aufbewahrungsbereich zum Aufbewahren von Lanzetten (1);
iii) einen Entnahmebereich zum Herausführen zumindest der Spitze (5) einer Lanzette (1) aus dem System während des Stechvorgangs; und
iv) eine Transporteinheit (18), die Lanzetten aus dem Aufbewahrungsbereich in den Entnahmebereich transportieren kann,
wobei das Antriebselement (6) so angeordnet ist, dass es im Entnahmebereich auf die dort befindliche Lanzette (1) zur Ausführung des Stechvorgangs einwirkt und diese so aus einer Ruheposition in eine Stechposition bewegt wird, und
wobei die Stechhilfe im Entnahmebereich eine zentrale Rückholvorrichtung (7) aufweist, die mit der Lanzette (1), die sich im Entnahmebereich befindet, während die Lanzette zur Ausführung des Stechvorgangs von der Stechposition in ihre Ruheposition bewegt wird, wechselwirkt, wobei die Antriebseinheit und die Rückholvorrichtung (7) unabhängig voneinander an der Lanzette (1) angreifen, **dadurch gekennzeichnet, dass** die Rückführung verschiedener Lanzetten (1) durch nur eine Rückholvorrichtung (7) bewirkt wird,

## Claims

1. System for storing and providing lancets for collecting body fluids comprising
i) a plurality of essentially needle-shaped lancets (1);
ii) a drive unit which has a drive element (6) which, in order to carry out a lancing process, is moved from a resting position into a lancing position;
iii) a storage area to store the lancets (1);
iv) a withdrawal area to guide at least the tip (5) of a lancet (1) out of the system during the lancing process and
v) a transport unit (18) which can transport lancets (1) from the storage area into the withdrawal area,
wherein the drive element (6) is arranged in such a manner that in the withdrawal area it acts on the lancet (1) located there to carry out a lancing process and thus moves the lancet from a resting position into a lancing position and wherein the system has a retraction device (7) in the withdrawal area which interacts with the lancet (1) which is located in the withdrawal area, while the lancet (1) is moved from the lancing position into its resting position in order to carry out the lancing process, wherein the drive unit and the retraction device (7) act independently of one another on the lancet (1) **characterized in that** the lancet (1) is returned by a central retraction device (7) such that different lancets are returned by only one retraction device.

2. System according to claim 1, **characterized in that** the end of the lancet opposite to the tip has an element that can be gripped by a retraction device.

3. System according to claim 1, **characterized in that** the return device contains a spring mechanism.

4. System according to one of the previous claims, **characterized in that** the lancets are stored in a magazine.

5. System according to claim 4, **characterized in that** the magazine contains the retraction device.

6. Lancet magazine (10) for storing lancets (1) comprising
i) a plurality of essentially needle-shaped lancets (1),
ii) a storage area for storing the lancets (1), and
iii) a withdrawal area for driving at least the tip (5) of a lancet out of the magazine (10) during the lancing process, which can be driven out by means of a drive unit;
wherein the magazine (10) has a central retraction device (7) in the withdrawal area which interacts with the lancet (1) located in the withdrawal area in such a manner that the retraction device (7) acts on the lancet (1) independently of a drive unit in order to move the lancet (1) from the lancing position into its resting position after the lancing process has been carried out, **characterized in that** different lancets are returned only by one retraction device.

7. Lancet magazine according to claim 6, additionally containing
iv) a transport unit which can transport lancets from the storage area into the withdrawal area.

8. Lancet magazine according to claim 6 or 7, **characterized in that** the end of the lancet opposite to the tip has an element that can be gripped by the retraction device.

9. Lancet magazine according to claim 6, 7 or 8, **characterized in that** the retraction device contains a spring mechanism.

10. Lancing device suitable for use in a system for storing and providing lancets (1) in a magazine comprising
i) a drive unit which has a drive element (6) that is moved from a resting position into a lancing position in order to carry out a lancing process;
ii) a storage area to store the lancets (1);
iii) a withdrawal area to guide at least the tip (5) of a lancet (1) out of the system during the lancing process; and
iv) a transport unit (18) which can transport lancets from the storage area into the withdrawal area,
wherein the drive element (6) is arranged in such a manner that in the withdrawal area it acts on the lancet (1) located there to carry out a lancing process and thus moves the lancet from its resting position into a lancing position and
wherein the lancing device has a central retraction device (7) in the withdrawal area which interacts with the lancet (1) located in the withdrawal area while the lancet is moved from the lancing position into its resting position in order to carry out the lancing process, wherein the drive unit and the retraction device (7) act independently of one another on the lancet (1) **characterized in that** different lancets (1) are returned by only one retraction device (7).

## Revendications

1. Système de stockage et de mise à disposition de lancettes pour l'extraction de fluides corporels comprenant :
i) une pluralité de lancettes (1) essentiellement en forme d'aiguille,
ii) une unité d'entraînement (6) qui comprend un élément d'entraînement, lequel se déplace d'une position de repos à une position de piquage pour exécuter un processus de piquage ;
iii) une zone de stockage pour le stockage de lancettes (1) ;
iv) une zone d'extraction pour mener au moins la pointe (5) d'une lancette (1) hors du système pendant le processus de piquage et une unité de transport (18) qui peut transporter les lancettes (1) de la zone de stockage à la zone d'extraction, et
l'élément d'entraînement (6) étant disposé de sorte à agir dans la zone d'extraction sur la lancette (1) y présente pour exécuter le processus de piquage et à ainsi déplacer celle-ci d'une position de repos à une position de piquage, et
le système comprenant dans la zone d'extraction un dispositif de rétraction (7) qui interagit avec la lancette (1) présente dans la zone d'extraction pendant que la lancette (1) est déplacée pour l'exécution du processus de piquage, de la position de piquage à sa position de repos, l'unité d'entraînement et le dispositif de rétraction (7) saisissant la lancette (1) indépendamment l'un de l'autre, **caractérisé en ce que** le retour en position de la lancette (1) est effectué au moyen d'un dispositif central de rétraction (7) de sorte que le retour en position de différentes lancettes soit effectué par un seul dispositif de rétraction.

2. Système selon la revendication 1, dans lequel la lancette possède à son extrémité opposée à la pointe un élément pouvant être saisi par le dispositif de rétraction.

3. Système selon la revendication 1, dans lequel le dispositif de rétraction comprend un mécanisme de ressort.

4. Système selon l'une des revendications précédentes, dans lequel les lancettes sont stockées dans un magasin.

5. Système selon la revendication 4, dans lequel est contenu, dans le dispositif de rétraction, le magasin.

6. Magasin de lancettes (10) pour le stockage de lancettes (1), comprenant
i) une pluralité de lancettes (1) essentiellement en forme d'aiguille,
ii) une zone de stockage pour le stockage de lancettes (1) ; et
iii) une zone d'extraction pour mener au moins la pointe (5) d'une lancette (1) hors du magasin (10) pendant le processus de piquage, laquelle peut être menée au moyen d'une unité d'entraînement,
le magasin (10) comprenant dans la zone d'extraction un dispositif central de rétraction (7) qui interagit avec la lancette (1) présente dans la zone d'extraction, de sorte que le dispositif de rétraction saisit la lancette (1) indépendamment d'une unité d'entraînement pour déplacer la lancette (1) de la position de piquage à sa position de repos après l'exécution du processus de piquage, **caractérisé en ce que** le retour en position de différentes lancettes est effectué par un seul dispositif de rétraction.

7. Magasin de lancettes selon la revendication 6, contenant en outre
iv) une unité de transport qui peut transporter les lancettes hors de la zone de stockage dans la zone d'extraction.

8. Magasin de lancettes selon la revendication 6 ou 7, dans lequel la lancette comporte à son extrémité opposée à la pointe un élément pouvant être saisi par le dispositif de rétraction.

9. Magasin de lancettes selon la revendication 6, 7 ou 8, dans lequel le dispositif de rétraction contient un mécanisme de ressort.

10. Dispositif de piquage approprié pour utilisation dans un système destiné au stockage et à la mise à disposition de lancettes dans un magasin comprenant
i) une unité d'entraînement qui comprend un élément d'entraînement (6), lequel est déplacé pour exécuter un processus de piquage hors d'une position de repos à une position de piquage;
ii) une zone de stockage pour le stockage de lancettes (1) ;
iii) une zone d'extraction pour mener au moins la pointe (5) d'une lancette (1) hors du système pendant le processus de piquage; et
iv) une unité de transport (18) qui peut transporter les lancettes hors de la zone de stockage dans la zone d'extraction,
l'élément d'entraînement (6) étant disposé de sorte à agir dans la zone d'extraction sur la lancette (1) y présente pour exécuter le processus de piquage et à ainsi déplacer celle-ci d'une position de repos à une position de piquage, et
le dispositif de piquage comprenant dans la zone d'extraction un dispositif central de rétraction (7) qui interagit avec la lancette (1) présente dans la zone d'extraction pendant que la lancette (1) est déplacée, pour l'exécution du processus de piquage, de la position de piquage à sa position de repos, l'unité d'entraînement et le dispositif de rétraction (7) saisissant la lancette (1) indépendamment l'un de l'autre, **caractérisé en ce que** le retour en position de différentes lancettes (1) est effectué par un seul dispositif de rétraction (7).
